# Europäisches Patentamt
# European Patent Office
# Office européen des brevets

(19)

(11) Veröffentlichungsnummer: **0 127 581**
**B1**

(12)

# EUROPÄISCHE PATENTSCHRIFT

(45) Veröffentlichungstag der Patentschrift:
**10.05.89**

(21) Anmeldenummer: **84810254.7**

(22) Anmeldetag: **24.05.84**

(51) Int. Cl.⁴: **C 12 N 1/20,** C 12 N 1/32,
C 02 F 3/34 //
C12R1/38

(54) **Mikroorganismen des Genus Pseudomonas und Verfahren zum Abbau von methylgruppenhaltigen Verbindungen in wässrigen Lösungen.**

(30) Priorität: **31.05.83 CH 2962/83**

(43) Veröffentlichungstag der Anmeldung:
**05.12.84 Patentblatt 84/49**

(45) Bekanntmachung des Hinweises auf die Patenterteilung:
**10.05.89 Patentblatt 89/19**

(84) Benannte Vertragsstaaten:
**AT BE CH DE FR GB IT LI LU NL SE**

(56) Entgegenhaltungen:
**EP-A-0 062 814**

**CHEMICAL ABSTRACTS, Band 91, Nr. 19, 5. November 1979, Seite 312, Nr. 153997j, Columbus, Ohio, US; M. BODZEK u.a.: "Dimethylamine and dimethylformamide as sources of carbon, nitrogen, and energy of microbes isolated from activated sludge" & GAZ, WODA TECH. SANIT. 1979, 53(4), 111-12**
**CHEMICAL ABSTRACTS, Band 98, Nr. 12, 21. März 1983, Seite 337, Nr. 95102v, Columbus, Ohio, US. H. BOSZCZYK-MALESZAK u.a.: "Treatment of sewage containing dimethylamine and dimethylformamide by activated sludge method. II. Influence of dimethylamine and dimethylformamide on respiration intensity and growth of microflora in the sludge" & GAZ, WODA TECH. SANIT. 1982, 56(5), 50-1**

(73) Patentinhaber: **CIBA-GEIGY AG, Klybeckstrasse 141, CH-4002 Basel (CH)**

(72) Erfinder: **Ghisalba, Oreste, Dr., Feldbergstrasse 91, CH-4057 Basel (CH)**
Erfinder: **Küenzi, Martin, Dr., Hüslimattstrasse 16, CH-4132 Muttenz (CH)**

EP 0 127 581 B1

**Beschreibung**

Die vorliegende Erfindung betrifft neue fakultative methylotrophe Mikroorganismen des Genus Pseudomonas oder eines Pseudomonas-ähnlichen Genus, Mischkulturen, ein Verfahren zur mikrobiologischen Reinigung von wässrigen Lösungen durch Abbau von Methylgruppenhaltigen Verbindungen in Gegenwart dieser Mikroorganismen, die Zellmasse mit ihren Bestandteilen, welche von diesen Mikroorganismen hergestellt wird, sowie die Verwendung der verfahrensgemäss erhältlichen Zellmasse.

Unter dem Begriff "methylotrophe Mikroorganismen" versteht man solche Mikroorganismen, die auf Nährmedien wachsen, welche als Kohlenstoffquelle Verbindungen mit nur einem Kohlenstoffatom, z. B. Methanol, oder mehreren Kohlenstoffatomen ohne direkte C-C-Bindung, z. B. Dimethylamin, enthalten [Patt, Cole und Hansen, International J. Systematic Bacteriology 26 (2), (1979) 226 - 229].

Unter dem Begriff "fakultativ methylotrophe Mikroorganismen" versteht man solche Mikroorganismen, die auf Nährmedien wachsen, welche als Kohlenstoffquelle Verbindungen mit nur einem Kohlenstoffatom, z. B. Methanol und/oder Verbindungen mit mehreren Kohlenstoffatomen mit oder ohne C-C-Bindung, z. B. Glucose oder Dimethylamin, enthalten.

In der Literatur sind fakultativ methylotrophe Mikroorganismen beschrieben, welche in wässriger Lösung eine oder mehrere Verbindungen aus der folgenden Gruppe von organischen Verbindungen abbauen bzw. als Kohlenstoff- oder als Kohlenstoff- und Stickstoffquelle verwerten können: Methan, Methanol, Äthanol, Acetate, z. B. Natriumacetat, Glucose, bestimmte Methylammoniumverbindungen, z. B. Methyl-, Äthyl- oder Trimethylammoniumchlorid, oder die freien Amine von diesen Salzen.

Solche Mikroorganismen sind in verschiedenen Stammsammlungen, z. B. in der American Type Culture Collection (ATCC), in der Deutschen Sammlung von Mikroorganismen (DSM) oder in der National Collection of Industrial Bacteria (NCIB), hinterlegt und in den von diesen Hinterlegungsstellen publizierten Katalogen aufgeführt.

Im Environmental Protection Agency (EPA) Report 2-79-163 von Dojlido J.R. vom Dez. 1979, sowie in der Kurzpublikation in Chem. Ind. 34 (1982) 750 (Biologische Entfernung von Stickstoffverbindungen aus Abwässern) wird die Möglichkeit einer Eliminierbarkeit von Dimethylformamid in wässrigen Lösungen mit Klärschlämmen erwähnt. In beiden Publikationen fehlt aber eine allgemeine Charakterisierung der Mikroorganismen, welche in diesen Klärschlämmen enthalten sind, und eine Charakterisierung von Mikroorganismen, welche Dimethylformamid abbauen können.

In der Literatur gibt es keine Angaben über den Fundort, die Isolierung, Identifizierung oder die Hinterlegungsstelle eines Dimethylformamid-abbauenden Mikroorganismus oder einer Mischkultur. Auch die genannten Literaturstellen enthalten keinen Hinweis, einen isolierten Mikroorganismus oder eine isolierte Mischkultur in einem mikrobiologischen Verfahren zur Reinigung von Abwasser enthaltend Dimethylformamid als Verunreinigung zu verwenden.

Bei der grosstechnischen Produktion in der chemischen Industrie fallen wässrige Lösungen, z. B. Abwässer, an, welche beispielsweise Dimethylformamid und/oder Formamid, Methyl- oder Äthylformamid, Acetamid, Formiat- oder Acetatsalze, Glucose, Methanol, Äthanol, Methyl-, Äthyl-, Dimethyl- oder Trimethylammoniumchlorid, Dimethylphosphit, Trimethylphosphit, Acetonitril oder Isobutyronitril als Verunreinigungen enthalten. Die Reinigung dieser Abwasser ist wegen der bisher üblichen Verbrennungsverfahren problematisch, weil die Verbrennung von organischen Abfallstoffen als äussert ungünstige Beseitigungsmethode mit grossen Kosten und ebenfalls hohen Belastungen für die Umwelt gilt.

Der vorliegenden Erfindung liegt die Aufgabe zugrunde, neue Mikroorganismen oder Mischkulturen zu finden und zu isolieren, welche in wässrigen Lösungen unter möglichst vollständigem Verbrauch der genannten Verbindungen ein besonders gutes Wachstum zeigen.

Diese Aufgabe wird durch die vorliegende Erfindung gelöst, welche neue fakultativ methylotrophe Mikroorganismen des Genus Pseudomonas oder eines Pseudomonas-ähnlichen Genus und Mischkulturen zum Gegenstand hat. Die vorliegende Erfindung hat ebenfalls ein Verfahren zur mikrobiologischen Reinigung von wässrigen Lösungen durch Abbau von Dimethylformamid, Formamid, Methyl- oder Äthylformamid, Acetamid, Methanol, Äthanol, Sacchariden, Niederalkanoaten, Methyl-, Äthyl-, Dimethyl- oder Trimethylammoniumhalogeniden, Dimethylphosphit, Trimethylphosphit, Hydantoin, Creatin, Creatinin, Barbitursäure, Cholin, Betain, Sarcosin, Dimethylglycin, Glycin, Gylyoxylsäure, N-Formylaminosäuren, N-Acetylaminosäuren, Acetonitril oder Isobutyronitril oder Mischungen dieser Verbindungen in Gegenwart dieser neuen Mikroorganismen oder Mischkultur, die Zellmasse mit ihren Bestandteilen, welche von diesen neuen Mikroorganismen oder Mischkultur hergestellt wird, sowie die Verwendung der verfahrensgemäss erhältlichen Zellmasse, zum Gegenstand.

Die weiter vorn und im folgenden verwendeten allgemeinen Begriffe haben in der Beschreibung der vorliegenden Erfindung vorzugsweise folgende Bedeutungen:

Saccharide sind z. B. Mono- oder Disaccharide z. B. Hexosen, z. B. Glucose oder Fructose.

Niederalkanoate sind z. B. Methyl- oder Äthylformiat oder -acetat oder Salze, z. B. Alkalimetallsalze, der Ameisensäure, z. B. Natrium- oder Kaliumformiat, oder Salze, z. B. Alkalimetallsalze, der Essigsäure, z. B. Natrium- oder Kaliumacetat.

Methyl-, Äthyl-, Dimethyl oder Trimethylammoniumhalogenide sind z. B. Methyl-, Äthyl-, Dimethyl- oder Trimethylammoniumchlorid, ferner -bromid.

N-Formylaminosäuren sind z. B. N-Formylglycin, -alanin, -valin, -serin oder -threonin.

2

N-Acetylaminosäuren sind z. B. N-Acetylglycin, -alanin, -valin, -serin oder -threonin.

Zellmasse sind z. B. alle sich in lebendem Zustand, z. B. im Teilungszustand oder Ruhezustand, im partiellen oder vollständigen Zelltod, oder bereits in der enzymatischen Zersetzung oder in der Zersetzung durch Fremdkulturen befindlichen Zellsysteme, welche von den Mikroorganismen der vorliegenden Anmeldung aufgebaut werden.

Bestandteile dieser Zellmasse sind z. B. die darin enthaltenen Enzyme bzw. die aus der Zellmasse erhältlichen Enzymextrakte, welche ebenfalls die weiter vorn genannten Verbindungen bzw. Verunreinigungen, z. B. Dimethylformamid, in wässriger Lösung abbauen können.

Die vorliegende Erfindung betrifft insbesondere fakultativ methylotrophe Mikroorganismen des Genus Pseudomonas oder eines Pseudomonas-ähnlichen Genus, Mischkulturen und ein Verfahren zur mikrobiologischen Reinigung von wässrigen Lösungen durch Abbau von Dimethylformamid, Methylformamid, Äthylformamid, Formamid, Methanol, Äthanol, Natrium- oder Kaliumformiat oder -acetat, Methyl-, Äthyl-, Dimethyl- oder Trimethylammoniumchlorid, Trimethylphosphit, Dimethylphosphit, Acetonitril, Isobutyronitril oder Mischungen dieser Verbindungen.

Die vorliegende Erfindung betrifft in erster Linie Mikroorganismen aus der Gruppe folgender Stämme: DMF 3/3 (NRRL-B-15358), DMF 3/4 (NRRL-B-15359), DMF 3/5 (NRRL-B-15360), DMF 3/6 (NRRL-B-15361), DMF 3/11 (NRRL-B-15362), DMF 3/12 (NRRL-B-15363), DMF 4/4 (NRRL-B-15364), DMF 5/3 (NRRL-B-15365), DMF 5/5 (NRRL-B-15366), DMF 5/7 (NRRL-B-15367), DMF 5/8 (NRRL-B-15368), DMF 5/9 (NRRL-B-15369), DMF 5/10 (NRRL-B-15370), Mischkulturen dieser Stämme, die Mischkultur DMF/HW 1-5 (NRRL-B-15371) und Verfahren zur mikrobiologischen Reinigung von wässrigen Lösungen durch Abbau von Dimethylformamid.

Die neuen Mikroorganismen stammen aus dem Klärschlamm einer Abwasserreinigungsanlage der Ciba-Geigy AG Schweiz (ARA Ciba-Geigy) und aus dem Klärschlamm eines Schlammteichs (Trockenbeet) in Kairouan, Tunesien. Die Mischkultur DMF/HW 1 - 5 wurde aus dem Sediment des Hallwilersees in der Schweiz isoliert. Sämtliche Mikroorganismen sowie die Mischkultur sind bei der Agricultural Research Culture Collection in Peoria, Illinois 61 604, USA am 13.4.83 hinterlegt worden.

In der folgenden Tabelle sind Hinterlegungsnummern und Fundort für jeden einzelnen Stamm bzw. für die Mischkultur angegeben:

**Tabelle 1:**

| Interne Bezeichnung | Hinterlegungsnummer | Fundort |
|---|---|---|
| DMF 3/3 | NRRL B-15358 | ARA Ciba-Geigy |
| DMF 3/4 | NRRL B-15359 | ARA Ciba-Geigy |
| DMF 3/5 | NRRL B-15360 | ARA Ciba-Geigy |
| DMF 3/6 | NRRL B-15361 | ARA Ciba-Geigy |
| DMF 3/11 | NRRL B-15362 | ARA Ciba-Geigy |
| DMF 3/12 | NRRL B-15363 | ARA Ciba-Geigy |
| DMF 4/4 | NRRL B-15364 | Kairouan |
| DMF 5/3 | NRRL B-15365 | Kairouan |
| DMF 5/5 | NRRL B-15366 | Kairouan |
| DMF 5/7 | NRRL B-15367 | Kairouan |
| DMF 5/8 | NRRL B-15368 | Kairouan |
| DMF 5/9 | NRRL B-15369 | Kairouan |
| DMF 5/10 | NRRL B-15370 | Kairouan |
| DMF/HW 1-5 (Mischkultur) | NRRL B-15371 | Hallwilersee |

**Charakterisierung der neuen Mikroorganismen:**

1. Isolierungsmethode:

Eine Abwasserprobe oder Klärschlammsuspension (1 g pro 10 ml sterilem Wasser) wurde in einen Schüttelkolben gegeben, worin sich 20 ml sterile Nährlösung MV 7 befand. Man gab ca. 0,1 g sterilfiltriertes Dimethylformamid hinzu, versetzte zur Pufferung der Nährlösung mit 1 ml M Phosphatpuffer von pH 7 und inkubierte bei 28° C die Schüttelkultur bei 250 Upm 7 - 14 Tage lang ml dieser ersten Anreicherungskultur wurde in 20 ml frische Nährlösung MV 7 mit 0,1 g Dimethylformamid gegeben und nochmals bei 28° und pH 7 (Schüttelkul tur 250 Upm, 7 - 14 Tage) inkubiert. 1 ml der zweiten Anreicherungskultur wurde wieder in 20 ml frische Nährlösung MV 7 mit 0,1 g Dimethylformamid gegeben und unter den genannten Bedingungen inkubiert. Aus 1 ml der dritten Anreicherungskultur stellte man nochmals mit 20 ml Nährlösung MV 7 und 0,1 g Dimethylformamid unter den genannten Bedingungen eine vierte Anreicherungskultur her. Die so hergestellten Anreicherungskulturen wurden jeweils auf sterile feste MV 7-Agar Nährböden [Nährlösung MV 7 mit Zusatz von 20 g/l Agar (Difco)] plattiert und bei 28°C inkubiert. Einzelkolonien wurden vorsichtig abgehoben und wieder auf dem gleichen Medium ausgestrichen. Diese Prozedur wurde mehrmals wiederholt, bis reine Isolate vorlagen.

Die verwendete Nährlösung MV 7 enthält in einem Liter Wasser die folgenden Bestandteile: 2 g $NH_4NO_3$ (Stickstoffquelle), 1,4 g $Na_2HPO_4$, 0,6 g $KH_2PO_4$ (Puffer und Phosphorquelle), 0,2 g $MgSO_4 \cdot H_2O$, 0,01 g $CaCl_2 \cdot 2 H_2O$, 0,001 g $FeSO_4 \cdot 7 H_2O$ und 1 ml Spurenelementlösung (bestehend aus je 20 mg/l $Na_2MoO_4 \cdot 2 H_2O$, $Na_2B_4O_7 \cdot 10 H_2O$, $MnSO_4 \cdot H_2O$ und $CuSO_4 \cdot 5 H_2O$). Zur Herstellung dieser Nährlösung löst man die Salze in destilliertem Wasser, bringt die Lösung mit verdünnter, wässriger Natriumhydroxid-Lösung unter pH-Kontrolle auf pH 7 und füllt diese mit destilliertem Wasser auf 1 Liter auf. Zur Herstellung des festen Nährbodens MV 7-Agar werden der Nährlösung MV 7 noch 20 g/l Agar (Difco) zugesetzt. Man sterilisiert im Autoklaven (2 at, 120° C).

2. Allgemeine Befunde und Mikroskopie

Alle in der Tabelle 1 aufgezählten Stämme sowie die Mischkultur wachsen unter aeroben Bedingungen bei Temperaturen bis ca. 37°, optimal bei 28° bis 30°. Die Mikroorganismen sind gramnegativ (Proben für Gram-Färbetest aus einer MV 7-Dimethylformamid-Kultur entnommen) und Oxidase-positiv (Proben für Test von Nutrient-Agar, MV 7-DMF-Agar und MV 7-DMF-Flüssigkultur entnommen). In flüssigen Kulturen, welche Dimethylformamid enthalten, beobachtet man die Neigung aller Stämme zu flockigem Wachstum.

In der mikroskopischen Abbildung (Lichtmikroskop) haben die Stämme DMF 3/3, 3/4, 3/5, 3/6, 3/11, 3/12 die Gestalt von länglichen Stäbchen und die Stämme DMF 4/4, 5/3, 5/5, 5/7, 5/8, 5/9 und 5/10 die Gestalt von kurzen Stäbchen oder ovalen Kugeln. Das elektronenmikroskopische Bild befindet sich in guter Übereinstimmung mit den lichtmikroskopischen Beobachtungen. Man erkennt vor allem zwei Zelltypen:

a) längliche, z.T. polar begeisselte Stäbchen von der Grösse 0,8 x 2 μ (Stämme DMF 3/3, 3/4, 3/5, 3/6, 3/11, 3/12),

b) kurze, z. T. polar begeisselte Stäbchen von der Grösse 0,7 x 1,1 μ (Stämme DMF 4/4, 5/3, 5/5, 5/7, 5/8, 5/9 und 5/10). Bei den Stämmen DMF 4/4 und 5/3 sind kurze, feine "Haare" auf der Zellwand zu erkennen.

3. Biochemische Charakterisierung und Klassifizierung der neuen Mikroorganismen

Zur allgemeinen biochemischen Charakterisierung und Klassifizierung der in Tabelle 1 aufgezählten Stämme werden zwei käufliche Testsysteme verwendet.

a) "Oxi/Ferm Tube" - Test (Roche)

Dieses Testsystem wird bei gramnegativen Stäbchen mit positiver Oxidasereaktion verwendet. Der jeweilige Test wird parallel je zweimal mit Impfmaterial des betreffenden Stamms abgenommen von Dimethylformamid - Agar und Nutrient - Agar durchgeführt. Beschreibung der genormten biochemischen Tests und experimentelle Ausführung siehe Vorschrift des Herstellers. Die Testresultate sind in Tabelle 2 zusammengestellt.

**Tabelle 2:** DMF-Stämme getestet und mit "Oxi/Ferm Tube" (48/28° C)

| Interne Bezeichnung | biochemischer Test | | | | | | | | |
|---|---|---|---|---|---|---|---|---|---|
| | anaerobe Dextrose-spaltung | Arginindi-hydrolase | $N_2$-Pro-duktion | $H_2S$-Bildung | Indol-Bildung | Xylose-spaltung | aerobe Dextrose-spaltung | Urease | Citratver-wertung |
| DMF 3/3 | - | - | + | - | - | + | + | - | ± |
| DMF 3/4 | - | - | + | - | - | + | + | ± | + |
| DMF 3/5 | - | - | + | - | - | + | ± | + | + |
| DMF 3/6 | - | - | -* | - | - | + | + | ± | + |
| DMF 3/11 | - | - | ±* | - | - | + | ± | - | ± |
| DMF 3/12 | - | - | + | - | - | + | ± | - | + |
| DMF 4/4 | - | - | + | + | - | ± | ± | - | ± |
| DMF 5/3 | ± | - | + | + | - | ± | - | + | + |
| DMF 5/5 | - | - | + | + | - | ± | - | - | ± |
| DMF 5/7 | - | - | + | + | - | - | - | - | - |
| DMF 5/8 | - | - | + | + | - | ± | ± | ± | ± |
| DMF 5/9 | - | - | + | + | - | - | - | - | + |
| DMF 5/10 | - | - | + | + | - | ± | ± | - | + |

+ beide Paralleltests positiv,
- beide Paralleltests negativ,
± ein Paralleltest positiv, ein Paralelltest negativ,
* im API -20 E-Test (Tabelle 4) positiv).

Aus den Befunden geht hervor, dass die Gruppe der Stämme DMF 3/3, 3/4, 3/5, 3/6 3/11 und 3/12 ($H_2$S-

Bildung eindeutig negativ und Xylosespaltung eindeutig positiv) biochemisch sich von der Gruppe der Stämme DMF 4/4, 5/3, 5/5, 5/7, 5/8, 5/9 und 5/10 unterscheidet (H₂S-Bildung eindeutig positiv und Xylosespaltung nur in einem Fall eindeutig negativ). Die biochemischen Unterschiede innerhalb der beiden Gruppen sind gering. Nach numerischer Auswertung der Testresultate von Tabelle 2 anhand der vom Hersteller gegebenen Vorschrift lassen sich die einzelnen Stämme aufgrund ihres jeweiligen Grades an Uebereinstimmung zusammenfassen und wie folgt taxonomisch annähernd klassifizieren:

**Tabelle 3:** Taxonomische Zuordnung aufgrund Numerischer Auswertung des "Oxi-Ferm Tube" Tests

| Interne Bezeichnung | Taxonomische Zuordnung |
|---|---|
| DMF 3/3 | Pseudomonas stutzeri |
| DMF 3/4 | Pseudomonas-ähnlich, Achromobacter sp. |
| DMF 3/5 | " |
| DMF 3/6 | " |
| DMF 3/11 | " |
| DMF 3/12 | " |
| DMF 4/4 | " |
| DMF 5/7 | Pseudomonas vesicularis |
| DMF 5/3 | keine Zuordnung |
| DMF 5/5 | " |
| DMF 5/8 | " |
| DMF 5/9 | " |
| DMF 5/10 | " |

b) "API 20 E"-Test

Dieses Testsystem wird speziell zur Erkennung von Enterobacteriaceen und allgemein zur Bestimmung von gramnegativen Bakterien verwendet. Die genormten biochemischen Tests und ihre Ausführung sind in der Anleitung des Herstellers beschrieben. Die Tests wurden ebenfalls parallel je zweimal mit Impfmaterial des betreffenden Stamms abgenommen von Dimethylformamid-Agar und Nutrient-Agar durchgeführt.

**Tabelle 4**: DMF Stämme getestet mit API 20 E (API-Laborsystem GmbH) (48 h, 28°C)

| Biochemischer Test | | DMF 3/3 | DMF 3/4 | DMF 3/5 | DMF 3/6 | DMF 3/11 | DMF 3/12 | DMF 4/4 | DMF 5/3 | DMF 5/5 | DMF 5/7 | DMF 5/8 | DMF 5/9 | DMF 5/10 |
|---|---|---|---|---|---|---|---|---|---|---|---|---|---|---|
| ONPG: | Hydrolyse durch β-Galactosidase | ± | + | + | + | + | + | + | + | ± | + | ± | - | + |
| ADH: | Arginindihydrolase | - | - | - | - | - | - | - | - | - | - | - | - | - |
| LDC: | Lysindecarboxylase | - | - | - | - | - | - | - | - | - | - | - | - | - |
| ODC: | Ornithindecarboxylase | - | - | - | - | - | - | - | - | - | - | - | - | - |
| CIT: | Citratverwertung | ± | ± | + | + | + | + | ± | + | ± | - | ± | ± | ± |
| H₂: | Thiosulfatspaltung | - | - | - | - | - | - | - | - | - | - | - | - | - |
| URE: | Urease | - | - | - | - | - | - | - | - | - | - | - | - | - |
| TDA: | Tryptophandesaminase | - | - | ± | ± | ± | ± | - | - | - | - | - | - | - |
| IND: | Tryptophanabbau Indolbildung | - | - | - | - | - | - | - | - | - | - | - | - | - |
| VP: | Acetointest | - | ± | ± | ± | ± | ± | ± | ± | ± | ± | ± | ± | ± |
| GEL: | Gelatineverflüssigung | - | - | - | + | - | - | + | ± | - | - | - | - | + |
| GLU: | Glucoseverwertung | - | - | - | ± | ± | - | - | - | - | - | - | - | - |
| MAN: | Mannitverwertung | - | - | - | - | - | - | - | - | - | - | - | - | - |
| INO: | Inositverwertung | - | - | - | - | - | - | - | - | - | - | - | - | - |
| SOR: | Sorbitverwertung | - | - | - | - | - | - | - | - | - | - | - | - | - |
| RHA: | Rhamnoserverwertung | - | - | - | - | - | - | - | - | - | - | - | - | - |
| SAC: | Saccharoseverwertung | - | - | - | - | - | - | - | - | - | - | - | - | - |
| MEL: | Melibioseverwertung | - | - | - | - | - | - | - | - | - | - | - | - | - |
| AMY: | Amygdalinverwertung | - | - | - | - | - | - | - | - | - | - | - | - | - |
| ARA: | L-Arabinose- | - | ± | - | - | - | - | - | - | - | - | - | - | - |

| | verwertung | | | | | | | | | | | | | |
|---|---|---|---|---|---|---|---|---|---|---|---|---|---|---|
| OX: | Oxidase | + | + | + | + | + | + | + | + | + | + | + | + | + |
| NO$_2$: | Nitrat- | + | + | + | + | + | + | + | + | + | + | + | + | + |
| N$_2$: | reduktion | + | + | + | + | + | + | + | + | + | + | + | + | + |
| CAT: | Katalase | + | + | + | + | + | + | + | + | + | + | + | + | + |

Die numerische Auswertung der Resultate des API 20 E-Tests nach der vom Hersteller gegebenen Vorschrift erlaubt keine eindeutigen taxonomischen Zuordnungen, so dass die Resultate gemäss Tabelle 4 nur zur biochemischen Charakterisierung der betreffenden Stämme verwendet werden können.

Die Stämme DMF 3/3, 3/4, 3/5, 3/6, 3/11 und 3/12 kann man anhand von morphologischen Merkmalen (polar begeisselte Stäbchen) und biochemischen Eigenschaften im Oxi-Ferm Test und API-Test als Pseudomonaden klassifizieren. Die Stämme DMF 4/4, 5/3, 5/5, 5/7, 5/8, 5/9 und 5/10 haben eine ovale Zellform (sehr kurze, begeisselte Stäbchen bis kugelförmige Gestalt). Wegen einiger Ähnlichkeiten im biochemischen Verhalten (Ausnahme: H$_2$S-Bildung im Oxi-Ferm Test) werden auch diese Stämme in der Beschreibung der vorliegenden Erfindung als Pseudomonaden bezeichnet, zumal ovale Zellformen auch bei anderen Pseudomonasstämmen beobachtet werden, z. B. Pseudomonas putida oder Pseudomonas ovalis.

### 4. Konservierung der Stämme

Für die Konservierung der Stämme gemäss Tabelle 1 eignen sich folgende Methoden:

a) Adsorption des Zellmaterials des betreffenden Stamms auf Glaskügelchen in Glycerinlösung und anschliessende Lagerung bei -20°C,

b) Aufbewahrung des Zellmaterials des betreffenden Stamms über Schrägagar und

c) Lyo-Ampullen. Die betreffende Kultur wird von der Nährlösung abzentrifugiert und die Zellmasse in 1/4 bis 1/3 Volumen-Teilen 15-%-iger Skim-Milk resuspendiert und lyophilisiert.

### 5. Abbaubare Verbindungen

In der Tabelle 6 ist eine Auswahl derjenigen Verbindungen (Kohlenstoffquellen) angegeben, die von den in der Tabelle genannten Stämmen bzw. der Mischkultur bei Zusatz einer Stickstoffquelle, z. B. Ammoniumnitrat, abgebaut werden können:

**Tabelle 6:**

| | | | | | Interne Bezeichnung | | | | | | | | | |
|---|---|---|---|---|---|---|---|---|---|---|---|---|---|---|
| C-Quellen | DMF 3/3 | DMF 3/4 | DMF 3/5 | DMF 3/6 | DMF 3/11 | DMF 3/12 | DMF 4/4 | DMF 5/3 | DMF 5/5 | DMF 5/7 | DMF 5/8 | DMF 5/9 | DMF 5/10 | DMF/HW 1-5 |
| Dimethylformamid (2 g/l) | + | + | + | + | + | + | + | + | + | + | + | + | + | + |
| Formamid (2 g/l) | - | - | ± | ± | ± | ± | + | + | + | + | + | + | + | + |
| Methylformamid (5 g/l) | + | + | + | + | + | + | ± | + | ± | + | + | + | + | + |
| Äthylformamid (5 g/l) | + | + | + | + | + | + | ± | + | + | + | + | + | + | + |
| Acetamid (5 g/l) | + | + | + | + | + | + | + | + | + | + | + | + | + | + |
| Methylformiat (3 g/l) | + | + | + | + | + | + | + | + | + | + | + | + | + | + |
| Natriumformiat (2 g/l) | ± | ± | ± | ± | ± | ± | + | + | + | + | + | + | + | ± |
| Natriumacetat (2 g/l) | + | + | + | + | + | + | + | + | + | + | + | + | + | ± |
| Glucose (5 g/l) | + | + | + | + | + | + | + | + | + | + | + | + | + | + |
| Methanol (2 g/l) | + | + | + | + | + | + | - | - | - | - | - | - | - | + |
| Äthanol (2 g/l) | + | ± | + | + | + | + | + | + | + | + | + | + | + | + |
| Methylammonium-chlorid (5 g/l) | + | + | + | + | + | + | + | + | + | + | + | + | + | + |
| Dimethylammonium-chlorid (5 g/l) | + | ± | ± | ± | ± | ± | ± | + | + | + | + | + | + | + |
| Trimethylammonium-chlorid (5 g/l) | + | + | + | + | + | + | + | + | + | + | + | + | + | + |
| Äthylammonium- | + | + | + | + | + | + | + | + | + | + | + | + | + | + |

chlorid (5 g/l)

| | | | | | | | | | | | | | | |
|---|---|---|---|---|---|---|---|---|---|---|---|---|---|---|
| Trimethylphosphit (2 g/l) | + | + | ± | + | + | + | - | - | - | - | - | - | - | + |
| Hydantoin | + | + | + | + | + | + | + | + | + | + | + | + | + | + |
| Creatin | + | + | + | + | + | + | + | + | + | + | + | + | + | + |
| Creatinin | + | + | + | + | + | + | + | + | + | + | + | + | + | + |
| Barbitursäure | + | + | + | + | + | + | + | + | + | + | + | + | + | + |
| Cholin | + | + | + | + | + | + | + | + | + | + | + | + | + | + |
| Betain | + | + | + | + | + | + | + | + | + | + | + | + | + | + |
| Dimethylglycin | + | + | + | + | + | + | + | + | + | + | + | + | + | + |
| Sarcosin | + | + | + | + | + | + | + | + | + | + | + | + | + | + |
| Glycin | + | + | + | + | + | + | + | + | + | + | + | + | + | + |
| Glyoxylsäure | + | + | + | + | + | + | + | + | + | + | + | + | + | + |
| Acetonitril | + | + | + | + | + | + | + | + | + | + | + | + | + | + |
| Isobutyronitril | + | + | + | + | + | + | + | + | + | + | + | + | + | + |

+: gutes Wachstum,
± mässiges Wachstum,
- kein oder nur geringes Wachstum)

Falls die zu reinigende wässrige Lösung eine der in Tabelle 6 genannten Stickstoff-haltigen Verbindungen enthält, z. B. Dimethylformamid, Methylformamid, Äthylformamid, Hydantoin, Creatinin, Creatin oder Barbitursäure, braucht in den meisten Fällen keine Stickstoffquelle hinzugesetzt werden.

Die experimentellen Befunde gemäss Tabelle 6 wurden nach Herstellen einer Schüttelkultur durch Impfen der betreffenden DMF-haltigen Vorkultur in Nährlösung MV 7 enthaltend die betreffenden C- bzw. C- und N-Quellen (Zusammensetzung weiter vorn beschrieben), Abpuffern auf pH 7, Inkubation innerhalb von 5 - 8 Tagen bei 28°C und 250 Upm und Messung der optischen Dichte $OD_{650}$ (mässiges Wachstum $OD_{650} < 0,15$, gutes Wachstum $OD_{650} > 0,15$) und gegebenenfalls Zugabe von Ammoniumchlorid ermittelt.

Von den in der Tabelle 1 aufgezählten Stämmen können sich spontan Mutanten (natürliche Mutanten) bilden, oder es lassen sich künstliche Mutanten herstellen, die ebenso wie die natürlichen Stämme die weiter vorn genannten Verbindungen und Salze, insbesondere Dimethylformamid, in wässriger Lösung abbauen können und Zellmasse produzieren. Solche Mutanten kann man chemisch, z. B. durch Behandlung mit gewissen Guanidinderivaten, z. B. N-Methyl-N'-nitro-N-nitrosoguanidin oder mit Alkalinitrit, z. B. Natriumnitrit, oder physikalisch, z. B. durch Ultraviolett-, Röntgen- oder radioaktive Strahlung, erzeugen.

Die erfindungsgemässen Mikroorganismen finden Anwendung in einem Verfahren zur mikrobiologischen Reinigung von wässrigen Lösungen enthaltend Dimethylformamid, Formamid, Methyl-, Äthylformamid, Acetamid, Methanol, Äthanol, Saccharide, Niederalkanoate, Methyl-, Äthyl-, Dimethyl- oder Trimethylammoniumhalogenide, Dimethylphosphit, Trimethylphosphit, Hydantoin, Creatin, Creatinin, Barbitursäure, Cholin, Betain, Sarcosin, Dimethylglycin, Glycin, Glyoxylsäure, N-Formylaminosäuren, N-Acetylaminosäuren, Acetonitril oder Isobutyronitril oder Mischungen dieser Verbindungen, dadurch gekennzeichnet, dass man in einer solchen wässrigen Lösung einen Mikroorganismus des Genus Pseudomonas oder eines Pseudomonas-ähnlichen Genus, welcher in dieser Lösung Zellmasse produziert, oder eine von diesem Mikroorganismus abgeleitete Mutante, welche ebenfalls diese Zellmasse zu produzieren vermag, in Gegenwart von essentiellen anorganischen Salzen und gegebenenfalls einer Stickstoffquelle bei ca. 20°C bis ca. 40°C und einem pH-Wert von ca. 4 bis ca. 7,5 unter aeroben Bedingungen züchtet und, wenn erwünscht, die erhältliche Zellmasse isoliert.

Bei der Züchtung oder Fermentation bauen die in der Tabelle 1 aufgezählten Stämme die in wässriger Lösung, z. B. in einem Abwasser, befindlichen Verunreinigungen, z. B. Dimethylformamid, Formamid, Methyl-, Äthylformamid, Methanol, Äthanol, Natriumacetat, Glucose, Methyl-, Äthyl-, Dimethyl- oder Trimethylammoniumchlorid, Dimethylphosphit, Trimethylphosphit, Acetonitril, Isobutyronitril oder Mischungen dieser Verbindungen bzw. Salze ab und verbrauchen Sauerstoff. Beim Abbauverfahren produzieren die Mikroorganismen Zellmasse, welche z. B. durch ein Vielfaches der ungefähren Summenformel $C_5H_9NO_x$ (x = 4 - 5) und einen Gehalt von ca. 37,0 % C, 6,2 % H und 9,6 % N (lyophilisiert) charakterisiert ist. Als weitere Fermentationsprodukte werden Kohlendioxid, sowie unter neutralen und sauren Bedingungen Ammoniumionen gebildet. Während der Fermentation ist der pH-Wert der zu reinigenden Lösung durch Zugabe von Pufferlösung, z. B. Phosphatpufferlösung, oder von wässrigen Basen, z. B. verdünnter, wässriger Natrium- oder Kaliumhydroxidlösung, auf Werte zwischen 4,0 und 7,5, bevorzugt ca. 6 bis 7, einzustellen.

Zur Reinigung von wässrigen Lösungen, welche Dimethylformamid enthalten, verwendet man vorzugsweise die Stämme DMF 3/3, 3/4, 3/5, 3/6, 3/11, 3/12, 4/4, 5/3, 5/5, 5/7, 5/8, 5/9, 5/10 und die Mischkultur DMF/HW 1-5. Zur Reinigung von wässrigen Lösungen, welche Formamid enthalten, verwendet man vorzugsweise die Stämme DMF 4/4, 5/3, 5/5, 5/7, 5/8, 5/9, 5/10 und die Mischkultur DMF/HW 1-5.

Für den Abbau von Formiatsalzen, z. B. Natriumformiat, eignen sich nur die Stämme DMF 4/4, 5/3, 5/5, 5/7, 5/8, 5/9 und 5/10, von Methanol nur die Stämme 3/3, 3/4, 3/5, 3/6, 3/11, 3/12 und die Mischkultur DMF/HW 1-5, von Dimethylphosphit und Trimethylphosphit nur die Stämme DMF 3/3, 3/4, 3/6, 3/11, 3/12 und die Mischkultur DMF/HW 1 - 5.

Die Züchtung erfolgt in Gegenwart von essentiellen anorganischen Salzen. Solche Salze sind beispielsweise

7

in Wasser lösliche Halogenide, z. B. Chloride, Carbonate, Sulfate oder Phosphate von Alkalimetallen, z. B. Natrium oder Kalium, Erdalkalimetallen, z. B. Calcium oder Magnesium, oder Übergangsmetallen, z. B. Eisen, Mangan, Molybdän, Kupfer oder Zink.

Bevorzugte essentielle anorganische Salze sind beispielsweise die in der Nährlösung MV 7 enthaltenen Salze, z. B. Dinatrium- oder Dikaliumhydrogenphosphat, Natrium- oder Kaliumdihydrogenphosphat, Magnesium- und Eisensulfat und Kalium- und Calciumchlorid. In geringen Mengen können noch Zink-, Mangan- und Kupfersulfat, Natriummolybdat und Borax zugesetzt werden.

Zur Reinigung von wässrigen Lösungen, welche beispielsweise Methanol, Äthanol, Acetat oder Glucose und sonst keine Stickstoff-haltigen Verbindungen enthalten, setzt man als Stickstoffquelle beispielsweise Aminosäuren, Peptide oder Proteine bzw. deren Abbauprodukte wie Pepton oder Trypton, Fleischextrakte, Mehle, z. B. von Mais, Weizen oder Bohnen, z. B. von der Sojabohne, Destillationsrückstände von der Alkoholherstellung, Hefeextrakte, Ammoniumsalze, z. B. Ammoniumchlorid, oder Nitrate, z. B. Kalium- oder Ammoniumnitrat, hinzu.

Einige der in Tabelle 1 genannten Stämme können zu Beginn der Fermentation nur nach Zugabe einer Stickstoffquelle wachsen, auch wenn in der wässrigen Lösung bereits eine abzubauende Stickstoffhaltige Verbindung vorhanden ist. So setzt man eine Stickstoffquelle hinzu, wenn man in wässrigen Lösungen Dimethylformamid in Gegenwart der Stämme DMF 5/5, 5/7, 5/8, 5/9 und 5/10 oder Formamid in Gegenwart der Stämme DMF 3/3, 3/4, 3/5, 3/6, 3/11 und 3/12 abbauen will.

Die Züchtung erfolgt unter aeroben Bedingungen, z. B. unter Sauerstoff- oder Luftzufuhr, und zwar unter Schütteln oder Rühren in Schüttelkolben oder Fermentern. Bei Verwendung der erfindungsgemässen Mirkoorganismen in Kläranlagen kann die Züchtung in offenen oder geschlossenen Klärbecken auch in Gegenwart von anderen Mikroorganismen erfolgen. Die Züchtung lässt sich in einem Temperaturbereich von ca. 25° bis ca. 35°C, bevorzugt bei ca. 27° bis ca. 28°C, durchführen.

Die Züchtung kann ansatzweise, z. B. durch ein- oder mehrmalige Zugabe von Nährlösung, oder kontinuierlich durch dauernde Zugabe von Nährlösung, erfolgen.

Vorzugsweise züchtet man in mehreren Stufen, indem man zunächst eine oder mehrere Vorkulturen, z. B. in einem flüssigen Nährmedium, z. B. MV 7, herstellt, welche man anschliessend in die eigentliche Hauptkultur überimpft. Eine Vorkultur lässt sich beispielsweise herstellen, indem man eine Probe mit Zellmaterial des betreffenden Mikroorganismus, der beispielsweise über Schrägagar aufbewart wird, in eine sterile Nährlösung, z. B. MV 7, mit einer geeigneten Kohlenstoffquelle, z. B. Dimethylformamid, überträgt und mehrere Tage bei 28°C inkubiert. Mit dieser ersten Vorkultur impft man frische Nährlösungen, z. B. MV 7, mit derselben Kohlenstoffquelle an und inkubiert nochmals mehrere Tage bei 28°C.

Man kann den Fermentationsverlauf durch Probenentnahme analytisch während der Fermentation verfolgen, z. B. durch Messung des pH-Wertes der Kultur oder der optischen Dichte, welche ein Mass für das Wachstum des jeweiligen Stamms ist, sowie gravimetrisch anhand des Trockengewichts der gebildeten Zellmasse.

Die Zellmasse ist ebenfalls Gegenstand der vorliegenden Erfindung. Diese lässt sich z. B. nach einem der zahlreichen in der Europäischen Patentschrift 0 010 243 beschriebenen Verfahren aufarbeiten und z. B. in Düngemittel überführen.

Die Zellmasse ist ein wertvoller Rohstoff, der eine definierte und reproduzierbare Zusammensetzung hat. Ungefähre Summenformel: $C_5H_9NO_x$ (x = 4 - 5); Gehalt (Lyophilisat): 37,0 %, C, 6,2 % H und 9,6 %. N.

Ebenfalls Gegenstand der vorliegenden Erfindung sind die in der Zellmasse enthaltenen Enzyme bzw. der aus der Zellmasse erhältliche Enzymextrakt. Enzyme lassen sich aus der Zellmasse in an sich bekannter Weise, z. B. wie in der Britischen Patentanmeldung 2 019 390 beschrieben, extrahieren.

Die verfahrensgemäss erhältliche Zellmasse kann als Einzellerprotein beispielsweise als Viehfutterzusatz verwendet werden. Die Zellmasse lässt sich auch beispielsweise als Suspension oder aufgearbeitet, z. B. nach Entwässerung oder Pasteurisierung, als Düngemittel verwenden. Man kann die Zellmasse auch als Ausgangsmaterial zur Herstellung von Biogas mit hohem Heizwert (Zusammensetzung ca. 70 % Methan, 29 % Kohlendioxid und 1 % Wasserstoff, Heizwert ca. 5500 - 6500 kcal/m³), beispielsweise durch anaerobe Vergärung in Faultürmen, verwenden. Der Rückstand (Faulschlamm) aus dem Herstellungsverfahren von Biogas ist ebenfalls ein hochwertiger Dünger, der im Vergleich zur ursprünglichen Zellmasse mit Stickstoff stark angereichert ist.

Die folgenden Beispiele illustrieren die vorliegende Erfindung. Die Temperaturangaben erfolgen in Grad Celsius.

**Beispiel 1** (Herstellung der Vorkultur):

Eine Probe des auf Schrägagar aufbewahrten Mikroorganismus DMF 3/6 wird in einen Schüttelkolben zu 20 ml Nährlösung MV 7 mit der weiter vorn angegebenen Zusammensetzung enthaltend Dimethylformamid in einer Konzentration von 5 g/l gegeben, und 72 Stunden bei 28° und 250 Upm inkubiert. 5 - 7 ml dieser ersten Vorkultur werden in einen zweiten Schüttelkolben gegeben, enthaltend 100 ml Nährlösung MV 7 (ohne Ammoniumnitrat) und Dimethylformamid in einer Konzentration von 5 g/l und 5 mMol Phosphatpuffer (pH 7), und 72 Stunden bei 28° und 250 Upm inkubiert.

**Beispiel 2:**

Analog Beispiel 1 lassen sich Vorkulturen der Stämme DMF 3/3, 3/4, 3/5, 3/6, 3/11, 3/12, 4/4, 5/3, 5/5, 5/7, 5/8, 5/9, 5/10 und der Mischkultur DMF/HW 1-5 herstellen. Zur Herstellung von Vorkulturen der Stämme DMF 5/5, 5/7, 5/8, 5/9 und 5/10 setzt man Ammoniumnitrat (2 g/l) als Stickstoffquelle hinzu.

**Beispiel 3:**

In einem Laborfermenter werden gegebenenfalls hitzesterilisierte (Sterilisation 20 Min. bei 120°) Nährlösung MV 7 (ohne Ammoniumnitrat) mit einer gegebenenfalls sterilfiltrierten, wässrigen Dimethylformamid-Lösung so vereinigt, dass ein ungefähres Arbeitsvolumen von 10 l mit einer Konzentration von ca. 5 g/l Dimethylformamid resultiert.

Man gibt eine Probe mit ca. 500 ml der zweiten Vorkultur des Stamms DMF 3/6 hinzu und hält im Fermenter folgende Bedingungen ein: pH-Wert von 7,0, welcher durch Zugabe jeweils von wässriger 0,1 N NaOH- bzw. 0,1 N HCl-Lösung konstant gehalten wird, Temperatur: 28°, Luftzufuhr 0,25 l/1 Min. und Rührgeschwindigkeit von 400 - 700 Upm.

Der Stamm wächst in dieser Lösung enthaltend Dimethylformamid als einzige Kohlenstoff- und Stickstoffquelle. Nach ca. 55 Stunden ist das eingesetzte Dimethylformamid abgebaut. Die gebildete Zellmasse liegt als Gemisch von Einzelzellen oder Aggregaten verschiedener Grösse vor, welche man durch Absetzen oder Zentrifugieren abtrennen kann.

**Beispiel 4:**

Analog Beispiel 3 lässt sich Dimethylformamid in wässriger Lösung abbauen, indem man in einem Laborfermenter Vorkulturen oder Mischkulturen der Stämme DMF 3/3, 3/4, 3/5, 3/6, 3/11, 3/12, 4/4, 5/3, 5/5, 5/7, 5/8, 5/9 und 5/10 oder die Mischkultur DMF/HW 1-5 züchtet. Bei Verwendung von Vorkulturen der Stämme DMF 5/5, 5/7, 5/8, 5/9 und 5/10 setzt man Ammoniumnitrat (2 g/l) als Stickstoffquelle hinzu.

**Beispiel 5:**

In einem Laborfermenter werden gegebenenfalls hitzesterilisierte (Sterilisation 20 Min. bei 120°) Nährlösung MV 7 (ohne Ammoniumnitrat) mit einer gegebenenfalls sterilfiltrierten Dimethylformamid-haltigen Abwasserlösung (Zusammensetzung: ca. 6,0 % Dimethylformamid, 7,0 % Methanol, 1,0 % Natriumsulfat, 5,4 % Natriumdimethylphosphat, sowie 3,6 % organische Bestandteile und 77 % Wasser) so vereinigt, dass ein ungefähres Arbeitsvolumen von 8 l mit einer Konzentration von ca. 5 g/l Dimethylformamid resultiert. Man gibt eine Probe mit ca. 500 ml der zweiten Vorkultur des Stamms DMF 3/6 hinzu und halt im Fermenter die im Beispiel 3 genannten Bedingungen ein. Der Abbau von Dimethylformamid und Methanol erfolgt innerhalb von ca. 100 Stunden.

**Beispiel 6:**

Analog Beispiel 5 lässt sich Dimethylformamid in gegebenenfalls sterilfiltrierten Abwasserlösungen abbauen, indem man in einem Laborfermenter Vorkulturen oder Mischkulturen der Stämme DMF 3/3, 3/4, 3/5, 3/6, 3/11, 3/12, 4/4, 5/3, 5/5, 5/7, 5/8, 5/9 und 5/10 oder die Mischkultur DMF/HW 1-5 züchtet. Bei Verwendung von Vorkulturen der Stämme DMF 5/5, 5/7, 5/8, 5/9 und 5/10 setzt man Ammoniumnitrat (2 g/l) als Stickstoffquelle hinzu.

**Patentansprüche** für die Vertragsstaaten: BE, CH, DE, FR, GB, IT, LI, LU, NL, SE

1. Fakultativ methylotrophe, DMF-abbauende Mikroorganismen des Genus Pseudomonas aus der Gruppe folgender Stämme: DMF 3/3 /NRRL-B-15358), DMF 3/4 (NRRL-B-153359), DMF 3/5 (NRRL-B-15360), DMF 3/6 (NRRL-B-15361), DMF 3/11 (NRRL-B-15362), DMF 3/12 (NRRL-B-15363), DMF 4/4 (NRRL-B-15364), DMF 5/3 (NRRL-B-15365), DMF 5/5 (NRRL-B-15366), DMF 5/7 (NRRL-B-15367), DMF 5/8 (NRRL-B-15368), DMF 5/9 (NRRL-B-15369), und DMF 5/10 (NRRL-B-15370), die Mischkultur DMF/HW 1-5 (NRRL-B-15371), eine von diesen Mikroorganismen abgeleitete Mutante oder Mischkulturen davon zur Reinigung industrieller Abwässer, dadurch gekennzeichnet, dass sie in wässriger Lösung Dimethylformamid, Formamid, Methyl- oder Äthylformamid, Acetamid, Methanol, Äthanol, Saccharide, Niederalkanoate, Methyl-, Äthyl-, Dimethyl- oder Trimethylammoniumhalogenide, Dimethylphosphit, Trimethylphosphit, Hydantoin, Creatin, Creatinin, Barbitursäure, Cholin, Betain, Sarcosin, Dimethylglycin, Glyoxylsäure, N-Formylaminosäure, N-Actylaminosäuren, Acetonitril oder Isobutyronitril oder Mischungen dieser Verbindungen unter Zunahme Zellmasse abbauen können.

2. Die Mischkultur DMF/HW 1-5 (NRRL-B-15371) gemäss Anspruch 1.

3. Verfahren zur mikrobiologischen Reinigung von wässrigen Lösungen enthaltend Dimethylformamid, Formamid, Methyl- oder Äthylformamid, Acetamid, Methanol, Äthanol, Saccharide, Niederalkanoate, Methyl-, Äthyl-, Dimethyl- oder Trimethylammoniumhalogenide, Dimethylphosphit, Trimethylphosphit, Hydantoin, Creatin, Creatinin, Barbitursäure, Cholin, Betain, Sarcosin, Dimethylglycin, Glycin, Glyoxylsäure, N-Formylaminosäuren, N-Acetylaminosäure, Acetonitril oder Isobutyronitril oder Mischungen dieser Verbindungen, dadurch gekennzeichnet, dass man in einer solchen wässrigen Lösung einen Mikroorganismus entsprechend Anspruch 1 in Gegenwart von essentiellen anorganischen Salzen und gegebenenfalls einer Stickstoffquelle bei ca. 20°C bis ca. 40°C und einem pH-Wert von ca. 4 bis ca. 7,5 unter aeroben Bedingungen züchtet und, wenn erwünscht, die gebildete Zellmasse isoliert.

4. Verfahren nach Anspruch 3 zur mikrobiologischen Reinigung von wässrigen Lösungen enthaltend Dimethylformamid, Formamid, Methanol, Äthanol, Natrium- oder Kaliumformiat oder -acetat, Methyl-, Äthyl-, Dimethyl- oder Trimethylammoniumchlorid, Dimethylphosphit, Trimethylphosphit, Acetonitril, Isobutyronitril oder Mischungen dieser Verbindungen, dadurch gekennzeichnet, dass man in einer solchen wässrigen Lösung einen Mikroorganismus entsprechend Anspruch 1 züchtet.

5. Verfahren nach Anspruch 3 zur mikrobiologischen Reinigung von wässrigen Lösung enthaltend Dimethylformamid, dadurch gekennzeichnet, dass man in einer wässrigen Lösung einen Mikroorganismus des Genus Pseudomonas, gemäss Anspruch 1, oder die Mischkultur, gemäss Anspruch 2, züchtet.

6. Verfahren nach einem der Ansprüche 3 - 5, dadurch gekennzeichnet, dass man die Züchtung bei 28°C durchführt.

7. Verfahren nach einem der Ansprüche 3 - 6, dadurch gekennzeichnet, dass man die Züchtung kontinuierlich durchführt.

**Patentansprüche** für den Vertragsstaat: AT

1. Verfahren zur mikrobiologischen Reinigung von wässrigen Lösungen enthaltend Dimethylformamid, Formamid, Methyl- oder Äthylformamid, Acetamid, Methanol, Äthanol, Saccharide, Niederalkanoate, Methyl-, Äthyl-, Dimethyl- oder Trimethylammoniumhalogenide, Dimethylphosphit, Trimethylphosphit, Hydantoin, Creatin, Creatinin, Barbitursäure, Cholin, Betain, Sarcosin, Dimethylglycin, Glycin, Glyoxylsäure, N-Formylaminosäuren, N-Acetylaminosäure, Acetonitril oder Isobutyronitril oder Mischungen dieser Verbindungen, dadurch gekennzeichnet, dass man in einer solchen wässrigen Lösung einen Mikroorganismus des Genus Pseudomonas aus der Gruppe folgender Stämme: DMF 3/3 /NRRL-B-15358), DMF 3/4 (NRRL-B-153359), DMF 3/5 (NRRL-B-15360), DMF 3/6 (NRRL-B-15361), DMF 3/11 (NRRL-B-15362), DMF 3/12 (NRRL-B-15363), DMF 4/4 (NRRL-B-15364), DMF 5/3 (NRRL-B-15365), DMF 5/5 (NRRL-B-15366), DMF 5/7 (NRRL-B-15367), DMF 5/8 (NRRL-B-15368), DMF 5/9 (NRRL-B-15369), und DMF 5/10 (NRRL-B-15370), oder eine von diesen Mikroorganismus abgeleitet Mutante oder Mischkulturen davon, in Gegenwart von essentiellen anorganischen Salzen und gegebenenfalls einer Stickstoffquelle bei ca. 20°C bis ca. 40°C und einem pH-Wert von ca. 4 bis ca. 7,5 unter aeroben Bedingungen züchtet und, wenn erwünscht, die gebildete Zellmasse isoliert.

2. Verfahren nach Anspruch 1, dadurch gekennzeichnet, dass man die Züchtung bei 28°C durchführt.

3. Verfahren nach einem der Ansprüche 1 - 2, dadurch gekennzeichnet, dass man die Züchtung kontinuierlich durchführt.

**Claims** for Contracting States: BE, CH, DE, FR, GB, IT, LI, LU, NL, SE

1. Facultatively methylotrophic, DMF-degrading microorganisms of the genus Pseudomonas selected from the group of the following strains: DMF 3/3 (NRRL-B-15358), DMF 3/4 (NRRL-B-15359), DMF 3/5 (NRRL-B-15360), DMF 3/6 (NRRL-B-15361), DMF 3/11 (NRRL-B-15362), DMF 3/12 (NRRL-B-15363), DMF 4/4 (NRRL-B-15364), DMF 5/3 (NRRL-B-15365), DMF 5/5 (NRRL-B-15366), DMF 5/7 (NRRL-B-15367), DMF 5/8 (NRRL-B-15368), DMF 5/9 (NRRL-B-15369) and DMF 5/10 (NRRL-B-15370), the mixed culture DMF/HW 1-5 (NRRL-B-15371), a mutant derived from these microorganisms, or mixed cultures thereof, for the purification of industrial waste waters, characterised in that, in aqueous solution, they are able to degrade dimethylformamide, formamide, methyl- or ethyl-formamide, acetamide, methanol, ethanol, saccharides, lower alkanoates, methyl-, ethyl-, dimethyl- or trimethylammonium halides, dimethyl phosphite, trimethyl phosphite, hydantoin, creatine, creatinine, barbituric acid, choline, betaine, sarcosine, dimethylglycine, glycine, glyoxylic acid, N-formylamino acids, N-acetylamino acids, acetonitrile or isobutyronitrile or mixtures of these compounds, with an increase in the biomass.

2. The mixed culture DMF/HW 1-5 (NRRL-B-15371) according to claim 1.

3. A process for the microbiological purification of aqueous solutions containing dimethylformamide, formamide, methyl- or ethyl-formamide, acetamide, methanol, ethanol, saccharides, lower alkanoates, methyl-, ethyl-, dimethyl- or trimethyl-ammonium halides, dimethyl phosphite, trimethyl phosphite, hydantoin, creatine, creatinine, barbituric acid, choline, betaine, sarcosine, dimethylglycine, glycine, glyoxylic acid, N-formylamino acids, N-acetylamino acids, acetonitrile or isobutyronitrile or mixtures of these compounds, characterised in that there is cultured in such an aqueous solution a microorganism according to claim 1, in the presence of essential inorganic salts and optionally a nitrogen source, at from approximately 20°C to approximately 40°C and at a pH of from approximately 4 to approximately 7.5, under aerobic conditions, and, if desired, the resulting biomass is isolated.

4. A process according to claim 3 for the microbiological purification of aqueous solutions containing dimethylformamide, formamide, methanol, ethanol, sodium or potassium formate or acetate, methyl-, ethyl-, dimethyl- or trimethyl-ammonium chloride, dimethyl phosphite, trimethyl phosphite, acetonitrile, isobutyronitrile or mixtures of these compounds, characterised in that there is cultured in such an aqueous solution a microorganism according to claim 1.

5. A process according to claim 3 for the microbiological purification of aqueous solutions containing dimethylformamide, characterised in that there is cultured in an aqueous solution a microorganism of the genus pseudomonas according to claim 1, or the mixed culture according to claim 2.

6. A process according to any one of claims 3 to 5, characterised in that culturing is carried out at 28°C.

7. A process according to any one of claims 3 to 6, characterised in that culturing is carried out continuously.


**Claims** for Contracting State: AT

1. A process for the microbiological purification of aqueous solutions containing dimethylformamide, formamide, methyl- or ethyl-formamide, acetamide, methanol, ethanol, saccharides, lower alkanoates, methyl-, ethyl-, dimethyl- or trimethyl-ammonium halides, dimethyl phosphite, trimethyl phosphite, hydantoin, creatine, creatinine, barbituric acid, choline, betaine, sarcosine, dimethylglycine, glycine, glyoxylic acid, N-formylamino acids, N-acetylamino acids, acetonitrile or isobutyronitrile or mixtures of these compounds, characterised in that there is cultured in such an aqueous solution a microorganism of the genus Pseudomonas selected from the group of the following strains: DMF 3/3 (NRRL-B-15358), DMF 3/4 (NRRL-B-15359), DMF 3/5 (NRRL-B-15360), DMF 3/6 (NRRL-B-15361), DMF 3/11 (NRRL-B-15362), DMF 3/12 (NRRL-B-15363), DMF 4/4 (NRRL-B-15364), DMF 5/3 (NRRL-B-15365), DMF 5/5 (NRRL-B-15366), DMF 5/7 (NRRL-B-15367), DMF 5/8 (NRRL-B-15368), DMF 5/9 (NRRL-B-15369) and DMF 5/10 (NRRL-B-15370), or a mutant derived from these microorganisms, or mixed cultures thereof, in the presence of essential inorganic salts and optionally a nitrogen source, at from approximately 20°C to approximately 40°C and at a pH of from approximately 4 to approximately 7.5, under aerobic conditions, and, if desired, the resulting biomass is isolated.

2. A process according to claim 1, characterised in that culturing is carried out at 28°C.

3. A process according to one of claims 1 and 2, characterised in that culturing is carried out continuously.


**Revendications** pour les Etats contractants: BE, CH, DE, FR, GB, IT, LI, LU, NL, SE

1. Micro-organismes dégradant le DMF facultativement méthylotropes, du genre Pseudomonas provenant du groupe des souches suivantes: DMF 3/3 (NRRL-B-15358), DMF 3/4 (NRRL-B-15359), DMF 3/5 (NRRL-B-15360), DMF 3/6 (NRRL-B-15361), DMF 3/11 (NRRL-B-15362), DMF 3/12 (NRRL-B-15363), DMF 4/4 (NRRL-B-15364), DMF 5/3 (NRRL-B-15365), DMF 5/5 (NRRL-B-15366), DMF 5/7 (NRRL-B-15367), DMF 5/8 (NRRL-B-15368), DMF 5/9 (NRRL-B-15369) et DMF 5/10 (NRRL-B-15370), la culture mixte DMF/HW 1-5 (NRRL-B-15371), un mutant dérivé de ces micro-organismes ou des cultures mixtes de ceux-ci destinés à l'épuration des eaux résiduaires industrielles, caractérisés en ce qu'ils peuvent dégrader en solution aqueuse, le diméthylformamide, le formamide, le méthyl- ou éthylformamide, l'acétamide, le méthanol, l'éthanol, les saccharides, les alcanoates inférieurs, les halogénures de méthyl-, éthyl-, diméthyl- ou triméthylammonium, le diméthylphosphite, le triméthylphosphite, l'hydantoïne, la créatine, la créatinine, l'acide barbiturique, la choline, la bétaïne, la sarcosine, la diméthylglycine, la glycine, l'acide glyoxylique, les N-formylamino-acides, les N-acétylamino-acides, l'acétonitrile ou l'isobutyronitrile ou des mélanges de ces composés avec accroissement de la masse cellulaire.

2. La culture mixte DMF/HW 1-5 (NRRL-B-15371) selon la revendication 1.

3. Procédé d'épuration microbiologique de solutions aqueuses contenant du diméthylformamide, du formamide, du méthyl- ou éthylformamide, de l'acétamide, du méthanol, de l'éthanol, des saccharides, des alcanoates inférieurs, des halogénures de méthyl-, éthyl-, diméthyl- ou triméthylammonium, du diméthylphosphite, du triméthylphosphite, de l'hydantoïne, de la créatine, de la créatinine, de l'acide barbiturique, de la choline, de la bétaïne, de la sarcosine, de la diméthylglycine, de la glycine, de l'acide glyoxylique, des N-formylamino-acides, des N-acétylamino-acides, de l'acétonitrile ou de l'isobutyronitrile ou des mélanges de ces composés, caractérisé en ce que l'on cultive dans une telle solution aqueuse un micro-organisme selon la revendication 1 en présence de sels minéraux essentiels et éventuellement d'une source d'azote à environ 20°C jusqu'à environ 40°C et à une valeur de pH d'environ 4 à environ 7,5 dans des conditions aérobies et, si on le désire, en ce que l'on isole la masse cellulaire formée.

4. Procédé selon la revendication 3 pour l'épuration microbiologique de solutions aqueuses contenant du diméthylformamide, du formamide, du méthanol, de l'éthanol, du formiate ou de l'acétate de sodium ou de potassium, des chlorures de méthyl-, éthyl-, diméthyl- ou triméthylammonium, du diméthylphosphite, du triméthylphosphite, de l'acétonitrile, de l'isobutyronitrile ou des mélanges de ces composés, caractérisé en ce que l'on cultive dans une telle solution aqueuse un micro-organisme selon la revendication 1.

5. Procédé selon la revendication 3 pour l'épuration microbiologique de solutions aqueuses contenant du diméthylformamide, caractérisé en ce que l'on cultive dans une solution aqueuse un micro-organisme du genre pseudomonas selon la revendication 1, ou la culture mixte selon la revendication 2.

6. Procédé selon l'une dés revendications 3 à 5, caractérisé en ce que l'on effectue la culture à 28°C.

7. Procédé selon l'une des revendications 3 à 6, caractérisé en ce que l'on effectue la culture de façon continue.

**Revendications** pour l'Etat contractant: AT

1. Procédé d'épuration microbiologique de solutions aqueuses contenant du diméthylformamide, du formamide, du méthyl- ou éthylformamide, de l'acétamide, du méthanol, de l'éthanol, des saccharides, des alcanoates inférieurs, des halogénures de méthyl-, éthyl-, diméthyl- ou triméthylammonium, du diméthylphosphite, du triméthylphosphite, de l'hydantoïne, de la créatine, de la créatinine, de l'acide barbiturique, de la choline, de la bétaïne, de la sarcosine, de la diméthylglycine, de la glycine, de l'acide glyoxylique, des N-formylamino-acides, des N-acétylamino-acides, de l'acétonitrile ou de l'isobutyronitrile ou des mélanges de ces composés, caractérisé en ce que l'on cultive dans une telle solution aqueuse un micro-organisme du genre Pseudomonas provenant du groupe des souches suivantes: DMF 3/3 (NRRL-B-15358), DMF 3/4 (NRRL-B-15359), DMF 3/5 (NRRL-B-15360), DMF 3/6 (NRRL-B-15361), DMF 3/11 (NRRL-B-15362), DMF 3/12 (NRRL-B-15363), DMF 4/4 (NRRL-B-15364), DMF 5/3 (NRRL-B-15365), DMF 5/5 (NRRL-B-15366), DMF 5/7 (NRRL-B-15367), DMF 5/8 (NRRL-B-15368), DMF 5/9 (NRRL-B-15369) et DMF 5/10 (NRRL-B-15370), ou un mutant dérivé de ces micro-organismes ou des cultures mixtes de ceux-ci, en présence de sels minéraux essentiels et éventuellement d'une source d'azote à une température d'environ 20°C jusqu'à environ 40°C et à une valeur de pH d'environ 4 à environ 7,5 dans des conditions aérobies et, si on le désire, en ce que l'on isole la masse cellulaire formée.

2. Procédé selon la revendication 1, caractérisé en ce que l'on effectue la culture à 28°C.

3. Procédé selon l'une des revendications 1 - 2, caractérisé en ce que l'on effectue la culture de façon continue.